# EUROPEAN PATENT APPLICATION

(11) **EP 4 548 849 A1**
(43) Date of publication of application: **07.05.2025**
(21) Application number: 23207412.0
(22) Date of filing: 02.11.2023
(51) Int. Cl.: A61B 6/00

(54) **SYSTEM AND METHOD FOR CONTROLLING AN X-RAY EXPOSURE OF A PATIENT**

(71) Applicant: Koninklijke Philips N.V., 5656 AG Eindhoven (NL)
(72) Inventor: JOCKEL, Sascha Andreas, Eindhoven (NL); SCHLUETER, Mathias, Eindhoven (NL); MENSER, Bernd, 5656AG Eindhoven (NL); SAALBACH, Axel, Eindhoven (NL)
(74) Representative: Philips Intellectual Property & Standards

(57) **Abstract**

The invention concerns a system for controlling an exposure of a patient generated from an X-ray device with a machine learning model, the system comprising one or more processors configured to: receive spatial sensor data from an image device, receive one or more exposure parameter from the X-ray device, provide the spatial sensor data and the exposure setting to the machine learning model, and obtain a prediction of the optimal exposure setting for the given X-ray imaging circumstances from the machine learning model based on an analysis of the received spatial sensor data. The invention further concern an X-ray system comprising the above system for controlling the exposure and a method for controlling the exposure of a patient generated by an X-ray device.

## Description

### FIELD OF THE INVENTION

The present invention relates to the field of radiographic imaging. In particular the invention relates to the field of patient individual adjustment of the X-ray exposure setting during an X-ray examination.

### BACKGROUND OF THE INVENTION

Choosing the right exposure parameters for an ideal X-ray image is still an issue even in the digital X-ray age. As in the analogue age, choosing the wrong exposure parameters that are not adapted to the individual acquisition circumstances and patient can result in poorly exposed X-ray images that are, for example, over- or underexposed. While fixed radiography systems offer automatic exposure control mechanisms that measure a cut off dose for an ideal exposure, free examinations require the user to manually adjust exposure parameters to reflect the actual environmental exposure conditions. There are several commissions which recommends comparing the actual exposure quality derived from the X-ray image (so called "exposure index") against an anatomy specific target exposure index to verify proper exposure of a patient's X-ray. A deviation from the target exposure index leads to poor image quality - due to over- or underexposure. Both affects bear diagnostic challenges and may be reasons for retake of the X-ray images. For examinations made without automatic exposure control solutions radiographer must adjust the exposure of the patient manually depending on the current environmental conditions. Hence, manual exposures come with their own huge set of challenges. Consequently, the image quality as well as the exposure conditions in this type of examination fluctuates significantly and overexposed X-ray images are quite common.

### SUMMARY OF THE INVENTION

There may therefore be a need for a system for adjusting, in particular controlling, an exposure of a patient in preparation of or during an image acquisition session or run which can maintain the exposure and related parameters, acquisition settings, and/or conditions. In particular, there exists a need for free X-ray examination wherein the user has to manually adjust the exposure parameters, to maintain the optimum exposure, etc.

An object of the invention is to provide a system for predicting, and based on the prediction controlling an exposure of a patient, an X-ray device comprising such system, and a method for controlling an exposure of a patient for an X-ray device adapted to the current scene and individual patient and which make suggestions for exposure settings, and/or predicts exposure parameters.

The object of the present invention is solved by the subject matter of the independent claims where further embodiments are incorporated in the dependent claims. It is understood that the following aspect of the invention equally applies to the method, the X-ray system, to the computer program element and to the computer readable medium. Accordingly, any feature, function, step and/or element described in the following with reference to one aspect of the present disclosure equally applies to any other aspect of the present disclosure.

According to a first aspect of the invention, a system for controlling an exposure of a patient generated from an X-ray device, in particular for controlling an X-ray device for generating an X-ray image of a patient using a machine learning model (ML model) is provided. The system comprises one or more processors configured to receive spatial sensor data from an image device and receive at least one exposure setting from an X-ray device. The one or more processors are further configured to provide the spatial sensor data and the exposure settings to the machine learning model. Furthermore, the one or more processor are configured to obtain a prediction of the optimal exposure setting for the given X-ray imaging circumstances from the machine learning model based on an analysis of the received spatial sensor data. Expressed differently, the system comprises one or more processors configured to obtain a prediction of a set of ideal X-ray exposure settings for the currently given acquisition circumstances and the current patient from a prediction model that previously got trained by machine learning techniques. The one or more processors are configured to obtain spatial image data representing the current scene in front of the image device, for example a multidimensional, in particular 3D sensor, such as camera, the currently set exposure settings of the X-ray system and/or additional data representing the target exposure for the given examination.

In the context of the present invention, the term "exposure" shall be understood to describe exposure of a patient to a certain amount of radiation. An exposure to high radiation levels can have a range of health risk effects which is limited during an X-ray acquisition/X-ray imaging with the optimal dose for the diagnostic task (ALARA principle)

In the context of the present invention, the term "exposure setting" shall be understood to describe any parameter or value which may be set in the X-ray device/in the X-ray system for adjusting the overall exposure of the patient during the X-ray imaging. On the other hand the term "exposure setting" comprises also any parameter or value which may be derived and/or calculated from respective exposure settings/parameters which are set in the X-ray system. In other words, the term "exposure setting" may understood to describe exposure control parameters, and/or exposure indicators. The exposure control parameters are used to set parameters of the X-ray device, for adjusting the exposure settings for generating an X-ray image. These exposure control parameters may be used for controlling an X-ray generator to produce the voltage for the X-ray tube to generate the X-ray radiation. The exposure indicators are values for monitoring the exposure in the digital radiography. The exposure indicators may be at least one of an exposure index, a target exposure index, a deviation index. The exposure index is calculated from the generated X-ray image, the target exposure index defines the exposure target value for the respective body part (anatomy) of the patient. The deviation index described the deviation between the target and the actual value of the exposure.

Under the term "target exposure index" shall be understood to describe a reference exposure when an image is properly exposed, in particular for a defined system and diagnostic task. In other words, the target exposure index may describe the expected value of the exposure index when the image receptor is properly exposed. The target exposure index may be set by the user, the equipment manufacturer or by the imaging center and is dependent on the body part, view, examination procedure, and imaging receptor. The target exposure index therefore offers the possibility of specifying the noise level that is diagnostically useful for a certain examination. The actual "exposure index" describes the actual values of the exposure achieved with the current X-ray exposure settings. The deviation index (DI) in turn indicates the deviation of the actual exposure index (EI) from its target exposure index (EIT) value and may be presented in the unit "exposure points". A DI of ±1 exposure points indicates an over/underexposure of +25% / -20% respectively. Accordingly, expressed in exposure points a DI ranging from 0 to +/- 2.9 indicates an optimal exposure, a DI ranging from +/-3 to +/-5.9 indicates slight under or overexposure, and a DI of less / more +/-6 indicates significant under or overexposure.

The differentiation between the various exposure settings will also be described with the different embodiments of the present disclosure.

In the context of the present invention, the term "optimal exposure setting" shall be understood to describe an ideal exposure setting which is in an optimal range providing a good image quality with a sufficient dose of radiation, which dose of radiation does not cause any health risk for the patient. In particular, ideal exposure settings or ideal exposure control parameters describe the best suitable exposure control parameters for the current circumstances (for example scene and patient) for obtaining an X-ray image with ideal exposure indicators, accordingly as close as possible at the target exposure index.

In other words, this invention describes a smart exposure control system for X-ray devices which proposes/predicts a (n) setting/controlling/adapting of exposure parameters adapted to the current scene and individual patient from an image device combined with information from an X-ray device/system. This means, the described system may be able, based on the patient's body shape, geometric information of the current scene including X-ray system components, the spatial relationship of the scene objects to each other and the currently used X-ray exposure settings, to predict the optimal/better exposure settings, for instance in comparison to the currently set exposure settings. The one or more processor may execute a set of instructions, which cause the processor to apply the machine learning model to the received input data from the spatial image device and the X-ray device to predict the optimal exposure setting. The machine learning model may be a neural network wherein a variety of learning rules may be applied, for example supervised learning, unsupervised learning, reinforce learning. For example, for providing and/or processing of image data (also in combination with additional information like patient demographics) convolutional neural networks (CNN) may be used for the prediction. CNN is just to be considered as an illustrative example architecture, but the utilized neural network architecture is not limited to it.

The smart exposure control system may at least receive the information of the image device and the X-ray device, such that the image device and the X-ray device may not be necessarily an integral part of the smart exposure control system itself. A practical realization may be that the smart exposure control system may at least be a part of an X-ray control system software. On the other hand at least the spatial image device may be part of the smart exposure control system such that a direct information processing is possible. Additionally/optionally the smart exposure control system and the spatial image device may be attachable to any X-ray device. The image device may be part of the smart exposure control system itself or may be an external image device wherein the information obtained by the image device are provided to the system in particular are provided to the processor for further processing. The image device may be a technically separate module that can communicate with the smart exposure control system via interfaces. However, it must at least make its image(s) available to the smart exposure control system or upon request from the smart exposure control system. Further, the smart exposure control system may be configured as a standalone module which could be attached/connected to an arbitrary X-ray device/X-ray system wherein the system may get the relevant X-ray system information and current exposure settings for the planned exposure from the attached/connected X-ray device/X-ray system. Moreover, the system may be a part of the X-ray device/X-ray system, such that the system comprises the spatial image device and the X-ray device.

The aim of the smart exposure control system is to improve an exposure index histogram for the exposures of the patient population examined in such a way that it matches a target exposure index best possible and thus yields a lower deviation index. Image burnout effects caused by overexposure or excessive image noise caused underexposure can get reduced by the prevailing smart exposure control system and the patient would get exposed to a clinically meaningful radiation dose.

The smart exposure control system gets triggered with the current information from the spatial image device and from the X-ray device. Further, the target exposure index and examination type for the current X-ray imaging circumstances are provided to the smart exposure control system. For example this information may be provided to the system by a user, for instance a user input to the exposure control system. The output of the smart exposure control system may then be a prediction of the optimal exposure setting for the current scene and individual patient. The predicted exposure setting may be used by the system to suggest and/or also to control/adjust the actual exposure settings for controlling the X-ray generator prior to the X-ray of the patient. An automated adjustment in the system may be marked visually so that the operator of the X-ray system knows that the current exposure setting for controlling the X-ray generator deviate from the default (predefined) exposure settings currently selected for the given examination from the database. Different options for the prediction may be possible, where these options are described in detail with the exemplary embodiments of the present disclosure. For instance, either the prediction system may predict the ideal kV and mAs values (or kV, mA and ms) for the current scene and the individual patient or it may predict the difference between the ideal exposure settings and the currently selected exposure settings. Further, the system may simply predict the expected exposure index for the X-ray imaging circumstances and consequently can calculate the deviation index from the target index stored in the database for the given examination / body part upfront the X-ray exposure. On the other hand, depending on the specific use and the desired level of automation of the system's exposure control mechanism, a combination of the different predictions is also possible.

According to an exemplary embodiment of the invention, the prediction of the optimal exposure setting may comprise a prediction of the most suitable exposure setting for a current scene and a current patient prior to the exposure of the patient. The prediction for an X-ray imaging process is carried out before the patient is exposed with the X-ray radiation, such that the optimal dose, the optimal exposure, for the patient can be achieved during the X-ray imaging of the patient. This allows to prevent retakes of an X-ray imaging due to non-optimal X-ray device settings.

Further, according to an additionally or optionally embodiment of the invention, the one or more processors are further configured to control the X-ray device based on the predicted optimal exposure setting. This means besides the prediction, which indicates to a user whether the optimal exposure may be reached or how it may be reached (by amending the setting of the X-ray device), the smart exposure control system may correct the exposure settings, if the prediction does not correspond to the optimal exposure settings. This may result in a smart exposure control system which is able to automatically adapt the exposure settings to the given X-ray circumstances.

According to an exemplary embodiment of the invention, the machine learning model may be trained using training data comprising at least one of spatial information from the spatial image device, spatial information from the X-ray device, patient examination information, exposure control related information, and after exposure information. Depending on the prediction embodiment the ML model may be trained with necessary inputs and with optional additional inputs. The optional inputs may be used to improve the prediction accuracy through additional information. In general, the spatial information for the ML model may be spatial sensor data received from the image device, for instance depth images (depth maps or point clouds) or RGB-D images. The spatial information from the X-ray device may be described as X-ray geo data, such as 3D positions and spatial relations of the individual X-ray components. The patient examination information may comprise a body part to be examined, guidelines for positioning the body part to be examined, and a target value for the respective body part to be examined, such as the target exposure index. The after exposure information may be understood as exposure indicators derived from the actual X-ray. The exposure indicator occurring with the actual exposure settings set in the X-ray device. Further, the after exposure information may comprise any information gained from the X-ray system or gained from an X-ray image from the examined body part. For instance, the X-ray image data may be used for training the machine learning model for calculating actual exposure index and deviation (index) from the target exposure index. Exposure control related information may be respective exposure control parameter, i.e. exposure settings such exposure control/generator control data such as tube voltage (kV), tube current time product (mAs), tube current (mA), heating current (mA), exposure time (s).

The model may be trained by numerous examinations providing a plurality of spatial information, examination related information, after exposure information and exposure control related information.

For instance, according to an embodiment of the invention, wherein the smart exposure control system may predict optimal exposure settings, the training data for the training process of the ML model may preferably comprise at least the spatial sensor data and the exposure control related information. The other input data for training of the ML model as mentioned hereinabove may be additionally used for the training for improving the training process. During the prediction process the smart exposure control system may be able, by using the trained ML model, to predict based on the received spatial sensor data from the image device the optimal exposure setting, for instance the ideal exposure control parameter, such as kV, mAs, mA, and/or s for the given circumstances.

According to another embodiment of the invention, wherein the smart exposure control system may predict via a recommendation for an adjustment of the exposure settings, the training data for the training process of the ML model may preferably comprise at least spatial sensor data, exposure indicators of the actual/current X-ray, such as the exposure index, and exposure related control information, for example the exposure control parameters. The smart exposure control system using such a trained ML model may be able to predict expected exposure indicators prior to an X-ray imaging based on at least the spatial sensor data, the target value for the current patient, and the currently set exposure control related information. Further, for the prediction of the expected exposure indicators the smart exposure control system may be configured to carry out further recommendation and/or calculations which will be elucidated in the further embodiments of this disclosure.

According to an exemplary embodiment of the invention, the pre-trained model could be expanded with an online learning method for the continuous improvement and adaption of the model to the usage conditions and usual examined patient population of the respective X-ray device/system. For refinement of the existing pre-trained model, the system according to the present disclosure saves the input parameters during an observation phase on the system or at the central storage location for later access. The observation phase may be variable but may not fall below a certain number of examinations per body part to ensure proper prediction performance. The data collected in the observation phase may be used to further train (or retrain) the pre-trained model (machine learning model) with the aim of further improving the smart exposure control system's the prediction capabilities.

According to an exemplary embodiment of the invention, the exposure setting may comprise at least one exposure control parameter of the X-ray device for performing an X-ray imaging of a patient. The spatial sensor data from the image device and/or the exposure setting currently set in the X-ray device may be received before the prediction of the optimal exposure setting. In this embodiment the exposure setting may define exposures control parameter of the X-ray device, for example X-ray generator settings, which may be set automatically or manually by a user of the X-ray device. On the other hand the current exposure setting may be used for predicting a specific exposure, for example the exposure setting may be used to calculate the expected exposure index and its deviation index from an optimal target exposure index for the current patient. According to this embodiment for the prediction of the optimal exposure parameter only the sensor data from the image device may be used. Additionally, the X-ray device settings may be used, such as X-ray system geometric data, and/or the examination information wherein each are received before actual X-ray imaging of the patient. The data from the image device and/or from the X-ray device is gained during normal daily use of the X-ray system and can be used for online learning/training of the model by the machine learning model.

According to an exemplary embodiment of the invention, the spatial sensor data may comprise information about a distance to points in a scene from a patient to the image device in the sensor's field of view. The spatial image device may be any applicable device which is configured and/or able to generate depth information about a scene in the visual field of the image device. In particular, the sensor data may be generated by a laser measurement system, and/or a time of flight system, and/or LIDAR system, and/or structured light system, and/or active/passive stereoscopic vision system. This list is not limiting and other sensor devices may be used which are suitable for generating depth and/or 2.5D or 3D representations. The image device may have a field of view which includes at least part of a field of view of the X-ray device, the imaging system, wherein the spatial image data is obtained before the X-ray imaging procedure with the patient and indicative of a distance that parts of the patient's exterior have towards the spatial image device.

According to an exemplary embodiment of the invention, the predicted optimal exposure setting may be an exposure control parameter of the X-ray device for controlling at least one of a tube voltage of the X-ray tube, a tube current, a tube current-time product, a heating current of the X-ray tube, or an exposure time. Therefore, a user may be enabled to directly adjust the exposure setting based on the prediction for carrying out an X-ray image with optimal/ideal exposure. The smart exposure control system may also be configured to predict more than one exposure control parameter. For instance, the smart exposure control system may be able to predict the direct exposure control parameters, or correction values which could be used for correcting the exposure control parameter to the optimal exposure control parameters. The predicted exposure control parameter may be presented to the user, such that the user may manually adjust the exposure. On the other hand the exposure settings, i.e. the exposure control parameters are predicted and set directly in the X-ray device. When the predicted optimal exposure settings (exposure control parameters) are set directly by the smart exposure control system the adjustment of the exposure control parameter in the X-ray device may be presented to the user. Hence, the user may only be informed about the adjustment according to the predicted exposure settings or not.

According to an exemplary embodiment of the invention, the prediction of the exposure setting may comprise the prediction of at least one exposure indicator calculated from the exposure setting currently set in the X-ray device, which exposure indicator may be at least an expected exposure index. The expected exposure index may describe an expected exposure when using the currently set exposure settings in the X-ray device under consideration of the given circumstances and patient. This embodiment may also be called an indirect prediction, as the optimal exposure settings may not be predicted directly. The optimal exposure setting may be derived from the exposure indicators via recommendation or calculation. This may enable a different approach for the exposure control parameter to predict, in the end, the optimal exposure setting for the X-ray device and the respective X-ray imaging circumstances.

Also a combination of the above mentioned different exposure predictions of the exposure settings may be possible.

According to an exemplary embodiment of the invention, the one or more processors may be further configured to calculate a deviation index for the current patient from the expected exposure index with the currently set exposure settings of the X-ray device and from the target exposure index for the given X-ray imaging circumstances. Further, one or more processors may be configured to recommend an adjustment of the exposure setting based on the calculated deviation index of the current patient. Hence, this may be the prediction of an expected deviation from the ideal exposure of the patient considering the exposure settings currently set in the X-ray device. The prediction may indicate the deviation of the current exposure settings from the ideal exposure settings. For example, the prediction of the deviation can be provided in, e.g., exposure points (plus or minus format). This allows the user to know by how many exposure points the exposure settings need to be adjusted in order to get closer to the target exposure index for the given scene and circumstances.

Further, the prediction of the deviation from the target exposure is generated prior to the exposure of the patient. Accordingly, the user may be able to adjust the exposure settings for the specific scene and the image of a patient prior to the X-ray imaging process.

For example, the machine learning model, the neural network, may be trained to predict the exposure index and/or the deviation from the target exposure index given the selected exposure settings, wherein the selected exposure setting may be exposure settings of the X-ray device for the current scene (including also the spatial image data) and the individual patient. For example, since the deviation index is linked to exposure points, the user can indirectly conclude by how many exposure points (with constant kV) the exposure parameters would need to be corrected in order to achieve a more optimal exposure for the current environmental condition. This approach of indicating just the expected deviation may particularly be useful when the training data consists mostly of badly exposed X-ray images with only a few well exposed X-ray image examples (with deviation index close to 0).

According to an exemplary embodiment of the invention, the calculation of the deviation to the target exposure may be carried out by the smart exposure control system based on the spatial sensor data and the currently set one or more exposure parameter, in particular the exposure control parameters, received prior to the X-ray imaging.

According to an exemplary embodiment of the invention, the one or more processor may be configured to store at least one or a plurality of the inputs of X-ray examination, such as spatial information from the spatial image device, spatial information from the X-ray device, patient examination information, exposure control related information, or after exposure information for a later training phase. The processor(s) may be configured to store at least one or more, or all of the above mentioned parameters or any combinations thereof. With this embodiment the smart exposure control system may be able to be trained after the initial training phase. Hence, the smart exposure control system and the ML model may be retrained with the information stored and/or used by the smart exposure control system for further improving the prediction of the optimal exposure settings.

According to an exemplary embodiment of the invention, the one or more processor may be further configured to use the predicted exposure settings for the X-ray imaging circumstances to retrain the machine learning model based on an iterative adaptation cycle over a time interval.

Additionally/Optionally the retraining may comprise an adjustment of one or more predefined exposure settings stored in a predefined exposure setting database which is in communication with the one or more processors.

For instance, in the database specific exposure and filter settings are stored for specific examinations and patient types (such as very large, large, small, thin patients). However, if it may be noticed, for example by a user, that the smart exposure control system may be systematically and on average off with the predefined exposure and filter settings, and for example often lead to overexposure, on may adjust these default exposure and filter settings of the database accordingly. Then it may be observed again. The adjustment of the predefined exposure setting may not necessarily be linked with a specific prediction for a patient. Instead of making a patient-specific prediction of the ideal exposure setting, the system could also adjust the predefined setting of the exposure parameter database based on the iterative adaption cycle ("Train", "Learn", "Observe") at longer time intervals. This embodiment may have the advantage that the exposure prediction may be adapted to the usage characteristics of the X-ray system and may be better suited to the workflow and patient population usually examined with the system. For example, if users would usually always use a too small source image distance, the initial factory settings tailored to a larger SID would tend to produce overexposed images. In this case, the system would adjust itself in the learning phase to reduce the predefined dose according to the distance usually used in order to avoid overexposure.

According to an exemplary embodiment of the invention, the one or more processor may be further configured to control at least one exposure parameter for setting the optimal exposure control parameter in the X-ray device predicted by the machine learning model. In other words, the processor may control the X-ray device such that the optimal exposure settings are set for the respective scene and patient. Accordingly, the method for controlling the exposure may comprise further steps such as controlling the one or more exposure parameter in the X-ray device based on the predicted optimal exposure settings, for setting the parameters of the X-ray device such that the optimal exposure control parameters are set for the respective scene and patient.

According to an exemplary embodiment of the invention, the prediction of the optimal exposure settings for the given X-ray imaging circumstances may comprise a calculation of a correction factor for the currently set one or more exposure settings in the X-ray device, wherein the calculation of the correction factor is based on the received spatial sensor data and the received and currently set exposure setting. Additionally or optionally the correction factor may be usable for correcting the currently set exposure settings to the optimal exposure settings. The previous embodiments of the present disclosure is such that the machine learning model suggests/predicts the X-ray system concrete exposure control parameters/settings that are ideal for the current environmental conditions and the individual patient (kV and mAs or kV, mA and ms), this embodiment may take a different approach. The system may also be designed to predict/propose to the user, e.g., as a request to correct the exposure by two exposure points downwards, as an indication, to better respond to the current environmental conditions and the individual patient. In other words, the system may predict a correction value/factor for achieving the optimal exposure, wherein both the correction value/factor and the to be reached optimal exposure are gained by inference of the pre-trained model based on the input data.

According to an exemplary embodiment of the invention, the machine learning model may be a pre-trained model which is continuously trained on the system during usage of the system. Accordingly, the system can be configured to adapt exposure settings to a given environmental situation and individual patient. The processor may be configured to store at least one of the current exposure parameter set in the X-ray device, the exposure parameter received during the X-ray imaging, spatial sensor data of the current scene, and spatial sensor data received during the X-ray imaging for a later training phase. In contrast thereto, the pre-trained model may also be iteratively adapted in repetitious learning cycles to continuously improve the quality of the predictions and to adapt the individual or usage behavior of the X-ray device/system.

According to an exemplary embodiment of the invention, the system may further comprise a storage, wherein the exposure setting from the X-ray device may be stored in the storage and/or wherein the spatial sensor data from the image device may be stored in the storage. Further, the storage may comprise data set for the training data of the machine learning model, wherein the storage is in communication with the processor for being able to provide the stored information to the machine learning model. The system may be able to provide the measured and calculated exposure indicators, actual and predicted exposure settings from and/or to the storage. Further the system may be able to save these input data for an upcoming training cycle or transfer them into a central storage location. In particular, the (one or more) processor is configured to store the received input data from the image device and the X-ray device in the storage. The processor may be further configured to store all relevant X-ray system device settings, predefined parameter and output values/parameter from each device to which the processor is in communication, such as the X-ray device and its components, the X-ray system, and the image device.

According to an exemplary embodiment of the invention, the image device may be a range image device comprising at least one of a RGB sensor, RGBD sensor, 3D camera, LIDAR, Laser Scanner (in combination with pan-tilt units), an Ultrasound, a 3D Camera by Triangulation, a 3D Camera by Structured Light, a 3D Camera by Time of Flight, a 3D Camera by Stereoscopic Vision (active or passive). Range imaging may refer to a collection of techniques that may be used to produce a depth image or 3D representation showing the spatial relations of points in the acquired 3D scene, e.g. distance of the points in a scene from a specific point, normally associated with the focal point of the image device. Resulting range or 3D images have pixel values that correspond to the distance in physical units. Amongst usual range imaging sensors, also RGB -> D approaches are coming up to provide range information of the imaged scene. A range image device may be mounted in the vicinity of the X-ray system aiming to acquire spatial image data of the patient in front of the detector at the time of X-ray exposure. The depth image contains distance information of the individual pixels from the cameras focal point. By using calibration and registration techniques the camera coordinate system can be mapped/linked/transferred to the X-ray coordinate system. The spatial relationships of various objects that live in the camera and X-ray coordinate system can be determined by the resulting common coordinate system and may be used for further calculations. Then it is possible to determine the distance the patient has from the radiation source, for example, and what area of the patient will get irradiated. The spatial relationship of a scene can be analyzed from such spatial image such as a depth map or 3d representation.

According to an exemplary embodiment of the invention, the smart exposure control system may further comprise a display configured to display a graphical representation of the predicted exposure settings to a user. Hence, a user can directly derive from the display the status of the exposure. For instance, the display may indicate the prediction of the optimal exposure settings, the expected exposure index, or deviation from the target exposure index such that the user is able to adjust and control the exposure and the X-ray device accordingly.

According to a second aspect of the present invention, an X-ray system is provided, which comprises the smart exposure control system according to any one of the embodiments described herein. The X-ray system may further comprise an X-ray device comprising an X-ray source and a detector. In particular the X-ray system may be configured to perform the method as described with a further aspect of the present invention. The X-ray system may comprise a generator, an X-ray tube, an X-ray detector and a control system for controlling the X-ray exposure and the X-ray image receptor. Further, the X-ray system may comprise at least one image device as mentioned with the different embodiments in this disclosure. The X-ray system must be able to provide the input parameters for the machine learning model as elucidated with the various embodiments as described herein.

According to a third aspect of the present invention, a method for controlling an X-ray exposure of a patient is described. The method for controlling an exposure of a patient is able to use the system for controlling the exposure of the patient as described with the embodiments of the present disclosure. The method comprises the steps of receiving spatial sensor data from an image device, receiving one or more exposure parameter from an X-ray device, providing the spatial sensor data and the one or more exposure parameter to a machine learning model, generating a prediction of an optimal exposure setting for the given X-ray imaging circumstances with the machine learning model based on the received spatial sensor data and/or the one or more exposure parameter.

The term "obtain a prediction of the optimal exposure settings" may be understood to describe that the machine learning model generates as an output a prediction of the optimal exposure control parameters/settings which should be used for the X-ray device for the current scene, circumstances and patient for the current planned X-ray imaging procedure.

Further, the method may comprise further steps which are adapted to perform the different predictions for achieving an optimal X-ray exposure as described with the embodiments of the system according to the present disclosure.

According to a further aspect of the present invention, a computer program or a computer program element is provided that is characterized by being adapted to execute the method steps of the method according to one of the preceding embodiments, on an appropriate system. The computer program may comprise instructions, which, when the program is executed by a computer, cause the computer to carry out the method of any one of the embodiments as described herein above. The computer program element might therefore be stored on a computer unit, which might also be part of an embodiment of the present invention. This computing unit may be adapted to perform or induce a performing of the steps of the method described above. The computing unit can be adapted to operate automatically and/or to execute the orders of a user. A computer program may be loaded into a working memory of a data processor. The data processor may thus be equipped to carry out the method of the invention. This exemplary embodiment of the invention covers both, a computer program that right from the beginning uses the invention and a computer program that by means of an update turns an existing program into a program that uses the invention. Further on, the computer program element might be able to provide all necessary steps to fulfill the procedure of an exemplary embodiment of the method as described above.

According to a further aspect of the present invention, a computer readable medium, such as a CD-ROM, is presented wherein the computer readable medium has a computer program element stored on it which computer program element is described by the preceding section. A computer program may be stored and/ or distributed on a suitable medium, such as an optical storage medium or a solid-state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. The computer-readable medium may comprise instructions which, when executed by a computer, cause the computer to carry out the method of any one of the embodiments as described herein.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

### BRIEF DESCRIPTION OF THE DRAWINGS

The aspects defined above, and further aspects of the present invention are apparent from the examples of embodiment to be described hereinafter and are explained with reference to the examples of embodiment. The invention will be described in more detail hereinafter with reference to examples of embodiment but to which the invention is not limited.
Fig 1 illustrates a system for controlling an exposure of a patient according to an exemplary embodiment of the invention.
Fig. 2 illustrates an interface showing different exposure parameters used in the system according to an exemplary embodiment of the invention.
Fig. 3 illustrates a depth image obtained by an image device of the system according to an exemplary embodiment of the invention.

### DETAILED DESCRIPTION OF EMBODIMENTS

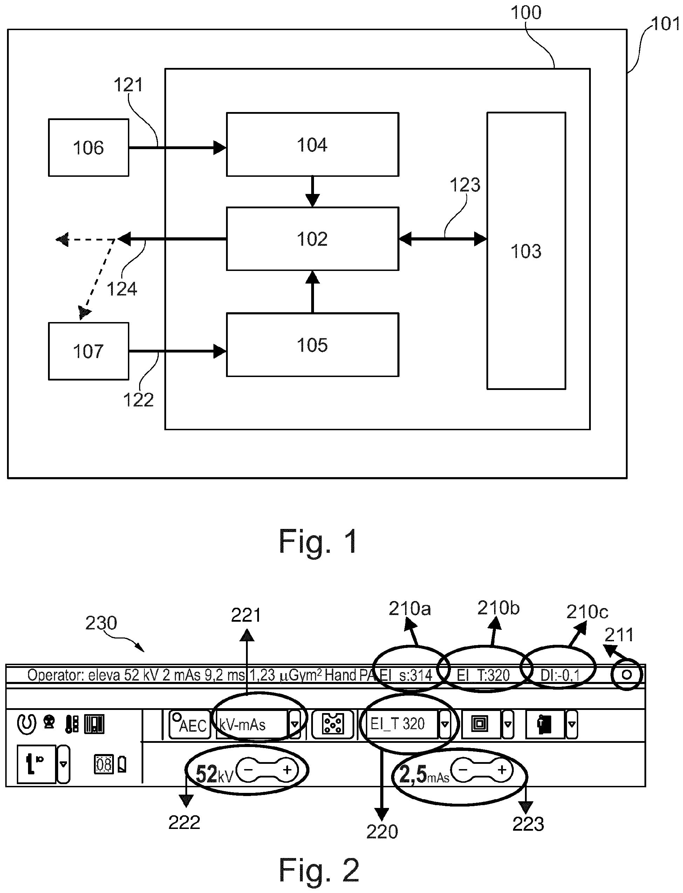

In Fig. 1 there is shown a system 100 for controlling an X-ray exposure of a patient generated from an X-ray device 107 with a smart exposure control system 100 based on a model trained by machine learning techniques, such as a machine learning model 103. The smart exposure control system 100 comprises one or more processors 102 configured to provide spatial sensor data received from a spatial image device 106 to the trained machine learning model 103 (which in this example would correspond to the use case of a trained model). The system 100 is further configured to provide one or more exposure settings, for example exposure control parameters received from the X-ray device 107 to the machine learning model 103 and to obtain a prediction 124 of the optimal exposure settings from the prediction model 103 based on the received spatial sensor data and/or the one or more exposure control parameters and X-ray system parameters. The prediction may be processed by the processor 102 and is afterwards provided to a prediction output 124 which may be able to provide the prediction to the operator of the X-ray system. As indicated in Fig. 1 with the dashed lines, the prediction output may be provided for instance to the X-ray device 107 (for instance for controlling the exposure of the X-ray device) or to any other prediction output which may be part of the X-ray system 101 or not. The system 100 for controlling the exposure may be part of an X-ray system 101, wherein the system 100 may be an external module attachable to the X-ray system 101 or the system 100 may be an integral part of the X-ray system 101. In particular, the system 100 may be a part of the X-ray device 107 of the X-ray system 101. As illustrated in Fig. 1 in the smart exposure control system 100 the machine learning model 103 is in communication with the processor 102, for example the processor 102 is configured to perform prediction or learning capabilities by carrying out several instructions. The processor 102 provides the spatial image data 104 and/or the X-ray data 105 to the machine learning algorithm 103. The X-ray data 105 may one or more of the following information: exposure control related information, examination related information, X-ray system geo data (for instance X-ray system related spatial information), or after exposure information. The system 100 may further comprise a storage, not illustrated, onto which the received image data 104 and X-ray data 105 may be stored, and which storage may be in communication with the machine learning model 103, for instance as a database for online and/or offline training. The spatial image data 104 is provided from a spatial image device 106. The X-ray image data 107 is provided from an X-ray device 107. The image device 106 and the X-ray device 107 according to this embodiment are associated with the X-ray system 101, in particular both elements 106, 107 are a part of the X-ray system 101. The machine learning model 103 may be trained using training data received from the image device 106, the image data 104, and received from the X-ray device 107, X-ray data 105. The training data may comprise at least one of spatial information from the spatial image device, spatial information from the X-ray device, patient examination information, exposure control related information, after exposure information. Examples of the training data may be the following, but may not be limited to this list: predefined exposure control parameters of the X-ray device 107, current exposure control parameters of the X-ray device 107, an actual exposure index, a target exposure index for the examined anatomy/body part and projection, a deviation index calculated from the deviation of the actual to the target exposure index, , 3-D sensor data, and RGB sensor data, X-ray image data 105, or data of the body part to be examined.

In Fig. 2 there is shown different exposure parameters used in the system 100 according to an exemplary embodiment of the invention. The different exposure parameters are indicated in an interface 230, which displays the exposure parameters to the user. The traffic light indicator 211 on the right side of Fig. 2 shows a color indication when for a given X-ray acquisition the actual exposure index deviates from the target exposure index to a certain extent, e.g., due to an under-/overexposure. For instance, the indicator 211 may be a visual light, showing a deviation in a certain color. The amount of the deviation may be color coded, such that a slight deviation may be indicated with a yellow light and a high deviation may be indicated by a red light. The exposure index 210a indicates the actual exposure of the X-ray and having the value of 314, the second exposure indicator 210b is the target exposure index of 320 and the third exposure indicator 210c is the deviation index of -0,1. The target exposure index may be adjustable 220 by the user. Further, the interface 230, i.e. display, is configured to display the exposure control parameters 222, 223 for controlling the X-ray device. The exposure control parameters 222, 223 are displayed in Fig. 2 with the values 52 kV and 2.5 mAs next to the plus minus buttons. Above the adjustable exposure control parameter a drop down menu 221 may be arranged as illustrated. With the drop down menu 221 the X-ray device, in particular the generator of the X-ray device may be controllable via the single values kV, mA, and/or ms. For example, if an exposure should be adjusted, hence controlled after an exposure prediction, a user may push the plus button 223 next to the mAs value for changing the exposure setting.

Accordingly, the interface 230 can indicate to the user whether the target exposure index is achieved and/or how much the actual exposure index 210a deviates from the target exposure index 210b. Further, the interface 230 may be able to display a prediction of the actual exposure index 210a prior to an X-ray imaging and could make suggestions on how to correct the actual exposure settings, for instance via the interface 230 by adjusting 52kV and/or 2,5mAs in Fig. 2, for reaching the optimal exposure close to the target exposure index.

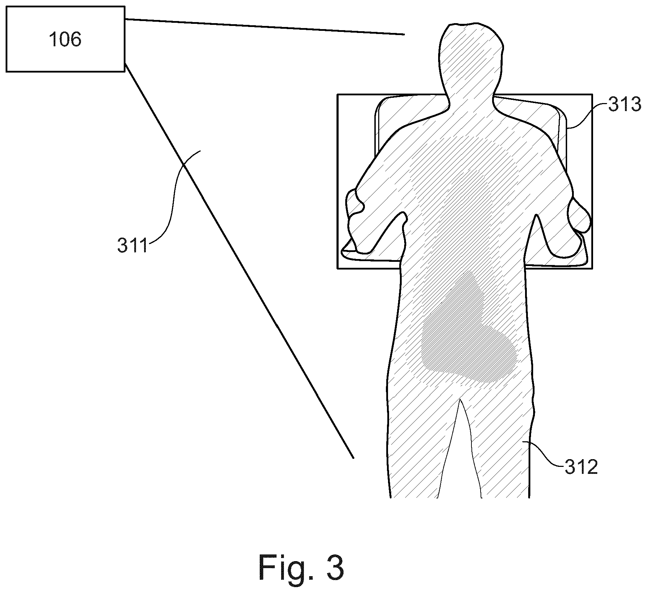

In Fig. 3 there is shown a depth map received from an image device 106. Hence the image data 104 may be a depth map which is derived from a field of view 311 viewed by the image device 106. In particular it is illustrated a depth map from a 3D sensor showing a patient 312 in front of an X-ray detector of a X-ray device 313 during an upright chest examination. The respective color (in Fig. 3 shown with different hatching of the lines, which in a real system would be displayed, for example, as pseudo-color or grayscale image) of each pixel corresponds to the distance to the camera. The denser the hatching in the illustrated depth map image of Fig. 3, the closer the pixels to the camera. The spatial relationships of a scene can be analyzed from such a depth map. A pseudo-color representation is just one form of representation of a 2.5 dimensional or 3 dimensional scene to make subtle nuances of different distances clearly distinguishable by using subtle nuances of a hue or shade of grey in an image. There are also numerous other forms of representation or just the grayscale image possible.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to their advantage. Any reference signs in the claims should not be construed as limiting the scope.

### LIST OF REFERENCE SIGNS:

- 100: smart exposure control system
- 101: X-ray system
- 102: processing unit
- 103: machine learning model
- 104: spatial image data
- 105: X-ray data
- 106: spatial image device
- 107: input X-ray device
- 121: image data communication
- 122: X-ray data communication
- 123: machine learning communication
- 124: prediction output
- 210: exposure indicators
- 211: deviation indication sign
- 220: target exposure index section box
- 221: dropdown menu
- 222: exposure control parameter
- 223: exposure control parameter
- 230: interface
- 311: field of view
- 312: patient
- 313: X-ray device

## Claims

1. A system (100) for controlling an X-ray exposure of a patient with a machine learning model (103), the system comprising one or more processors (102) configured to:
receive spatial sensor data from an image device (106);
receive at least one exposure setting from an X-ray device (107);
provide the spatial sensor data and the exposure settings to the machine learning model (103),
obtain a prediction of an optimal exposure setting for the given X-ray imaging circumstances from the machine learning model based on an analysis of the received spatial sensor data.

2. The system according to claim 1,
wherein the prediction of the optimal exposure setting comprises a prediction of the most suitable exposure setting for a current scene and a current patient prior to the exposure of the patient,
wherein the one or more processors (102) are further configured to control the X-ray device (107) based on the predicted optimal exposure setting.

3. The system according to claim 1 or 2,
wherein the machine learning model (103) is trained using training data comprising at least one of spatial information from the spatial image device, spatial information from the X-ray device, patient examination information, exposure control related information, and after exposure information.

4. The system according to any of the preceding claims,
wherein the received exposure setting comprises at least one exposure control parameter of the X-ray device (107) for performing an X-imaging of a patient,
wherein the spatial sensor data from the image device (106) and/or the exposure setting currently set in the X-ray device is received before the prediction of the optimal exposure setting.

5. The system according to claim according to any of the preceding claims,
wherein the spatial sensor data comprises information about a distance to points in a scene from a patient to the image device (106) in the field of view of the image device (106).

6. The system according to any of the preceding claims,
wherein the predicted optimal exposure setting is an exposure control parameter of the X-ray device (107) for controlling at least one of a tube voltage of the X-ray tube, a tube current, a tube current-time product, a heating current of the X-ray tube, or an exposure time.

7. The system according to any of the preceding claims,
wherein the prediction of the exposure setting comprises the prediction of at least one exposure indicator calculated from the currently set exposure setting in the X-ray device, which exposure indicator is at least one of an expected exposure index.

8. The system according to claim 7, wherein the one or more processors is further configured to
calculate a deviation index for the current patient from the expected exposure index with the currently set exposure settings of the X-ray device (107) and from the target exposure index for the given X-ray imaging circumstances,
recommend an adjustment of the exposure setting based on the calculated deviation index of the current patient.

9. The system according to any of the preceding claims,
wherein the one or more processor (102) is configured to store at least one or a plurality of the inputs of X-ray examination, such as spatial information from the spatial image device, spatial information from the X-ray device, patient examination information, exposure control related information, after exposure information for a later training phase.

10. The system according to any of the preceding claims,
wherein the one or more processor (102) is further configured to use the predicted exposure settings for the X-ray imaging circumstances to retrain the machine learning model based on an iterative adaptation cycle over a time interval,
wherein the retraining comprises an adjustment of one or more predefined exposure settings stored in a predefined exposure setting database which is in communication with the one or more processors.

11. The system according to any of the preceding claims,
wherein the prediction of the optimal exposure setting for the given X-ray imaging circumstances comprises a calculation of a correction factor for the currently set one or more exposure setting in the X-ray device, wherein the calculation of the correction factor is based on the received spatial sensor data and the received exposure setting,
wherein the correction factor is usable for correcting the currently set exposure setting to the optimal exposure setting.

12. The system according to any of the preceding claims, further comprising
a storage, wherein the exposure setting from the X-ray device is stored in the storage,
wherein the spatial sensor data from the image device is stored in the storage,
wherein data sets for the training data of the machine learning model is stored in the storage,
wherein the storage is in communication with the processor for being able to provide the stored information to the machine learning model.

13. The system according to any of the preceding claims, further comprising
a display configured to display a graphical representation of the predicted exposure settings to a user.

14. An X-ray system (101) comprising
an X-ray device (107) configured to perform an X-ray imaging process at a patient,
an image device (106) configured to obtain spatial sensor data of the patient and of a current scene with the patient,
a system (100) for controlling at least one exposure setting of the X-ray device (107) according to any of the preceding claims.

15. A computer implemented method of controlling an exposure of a patient, the method comprising the steps of
receiving spatial sensor data from an image device (106),
receiving one or more exposure setting from an X-ray device (107),
providing the spatial sensor data and the one or more exposure setting to a machine learning model,
generating a prediction of an optimal exposure setting for the given X-ray imaging circumstances with the machine learning model based on an analysis of the received spatial sensor data.
